# EUROPEAN PATENT APPLICATION

(11) **EP 2 072 049 A2**
(43) Date of publication of application: **24.06.2009**
(21) Application number: 09004205.2
(22) Date of filing: 20.06.2001
(51) Int. Cl.: A61K 31/41, A61K 9/20

(54) **Solid valsartan pharmaceutical compositions**

(30) Priority: 22.06.2000 US 599687
(62) Divisional of application: 01965014.2
(71) Applicant: Novartis AG, 4056 Basel (CH); Novartis Pharma AG, 1230 Vienna (AT)
(72) Inventor: Ganter, Sabina Maria, 79365 Rheinhausen (DE); Wagner, Robert Frank, Hillsborough NJ 08844 (US)
(74) Representative: Breuer, Markus

(57) **Abstract**

The invention relates to solid oral dosage forms comprising pharmacologically effective amounts of valsartan or a pharmaceutically acceptable salt thereof or hydrate thereof. The compositions are at least 1.2 times more bioavailable than conventional valsartan capsule.

## Description

This invention relates to solid oral dosage forms containing valsartan.

The angiotensin II receptor antagonist - valsartan - is known to be effective in the treatment of congestive heart failure and reducing blood pressure irrespective of age, sex or race and is also well tolerated. Its combination with HCTZ is also known for the treatment of hypertension.

WO 87/49394 - the content of which is incorporated herein by reference, especially (but not limited to) the subject matter as claimed - discloses compressed solid oral dosage forms, e.g. by compaction, of valsartan, optionally in salt form, optionally combined with hydrochlorothiazide (HCTZ).

It has been found surprisingly that it is possible to manufacture solid formulations (thereafter "compositions of the invention") of valsartan or a pharmaceutically acceptable salt thereof or hydrate thereof (thereafter "active agent") having improved bioavailability characteristics when compared to known valsartan formulation.

In a first aspect the present invention relates to an oral solid pharmaceutical composition, e.g. in form of a tablet, comprising pharmacologically effective amounts of valsartan or a pharmaceutically acceptable salt thereof or hydrate thereof and at least one pharmaceutical acceptable excipient and which is, on average, at least 1.2 times, e.g. 1.2 to 3 times, e.g. 1.3 to 2 times, e.g. 1.7 times more bioavailable than a valsartan capsule, e.g. as a capsule marketed under the trade name Diovan®, e.g. containing 20, 40, 80, or 160 mg of valsartan or any corresponding capsule containing a unit dose of 1 to 500 mg of valsartan.

In one embodiment, the present invention relates to an oral solid pharmaceutical composition, e.g. in form of a tablet, comprising pharmacologically effective amounts of valsartan or a pharmaceutically acceptable salt thereof or hydrate thereof and at least one pharmaceutically acceptable excipient and which is, on average, at least 1.5 times, e.g. up to 3 times, e.g. 2.5 times, more bioavailable than a valsartan capsule, e.g. as a capsule marketed under the trade name Diovan®, e.g. containing 20, 40, 80, or 160 mg of valsartan or any corresponding capsule containing a unit dose of 1 to 500 mg of valsartan, when administered as a dose of 40 mg in a single dose human bioavailability study.

In particular, the present invention relates to an oral solid pharmaceutical composition, e.g. in form of a tablet, comprising pharmacologically effective amounts of valsartan or a pharmaceutically acceptable salt thereof or hydrate thereof and at least one pharmaceutically acceptable excipient and which is, on average. at least 1.2 times, e.g. up to 2 times, e.g. 1.5 times, more bioavailable than a valsartan capsule, e.g. as a capsule marketed under the trade name Diovan®, e.g. containing 20, 40, 80, or 160 mg of valsartan or any corresponding capsule containing a unit dose of 1 to 500 mg of valsartan, when administered as a dose of 320 mg in a single dose human bioavailability study.

In a further aspect the present invention relates to an oral solid pharmaceutical composition, e.g. in form of a tablet, comprising pharmacologically effective amounts of valsartan or a pharmaceutically acceptable salt thereof or hydrate thereof and at least one pharmaceutically acceptable excipient and having an AUC higher than 4.5 h.mg/l, e.g. up to 8 h.mg/l, e.g. 6 h.mg/l, at same conditions as a 40 mg capsule has an AUC of 3.9 h.mg/l. If not indicated otherwise, the AUC values mentioned in this application are least square means.

In a further aspect the present invention relates to an oral solid pharmaceutical composition, e.g. in form of a tablet, comprising pharmacologically effective amounts of valsartan or a pharmaceutically acceptable salt thereof or hydrate thereof and at least one pharmaceutically acceptable excipient and having an AUC higher than 30 h.mg/l, e.g. up to 40 h.mg/l, e.g. 35 h.mg/l, at same conditions as a 320 mg capsule has an AUC of 29.4 h.mg/l.

In a further aspect the present invention relates to an oral solid pharmaceutical composition, e.g. in form of a tablet, comprising pharmacologically effective amounts of valsartan or a pharmaceutically acceptable salt thereof or hydrate thereof and at least one pharmaceutically acceptable excipient and having a Cmax of about at least 0.77 mg/l, e.g. up to 3.5 mg/l, e.g. 1.3 mg/l when administered as a dose of 40 mg in a single dose human bioavailability study.

In a further aspect the present invention relates to an oral solid pharmaceutical composition, e.g. in form of a tablet, comprising pharmacologically effective amounts of valsartan or a pharmaceutically acceptable salt thereof or hydrate thereof and at least one pharmaceutically acceptable excipient and having a Cmax of about at least 4.75 mg/l, e.g. up to 15 mg/l, e.g. 6 mg/l, when administered as a dose of 320 mg in a single dose human bioavailability study.

The compositions of the invention may comprise in a unit dose 1 to 500 mg, e.g. 2 to 400 mg, e.g. 5 to 300 mg, e.g. 10 to 200 mg, e.g. 20 to 200 mg, e.g. 30 to 100 mg, of active agent. Examples of doses are 10, 20, 40, 80, 160, or 320 mg.

Among other advantages, the increased bioavailability allows the manufacture of low dose solid oral dosage forms of active agent which are better tolerated by patients. The present invention thus relates in a further aspect to oral solid pharmaceutical compositions, e.g. in form of a tablet, of valsartan or a pharmaceutically acceptable salt thereof or hydrate thereof comprising less than 20 mg, e.g. 1 to 15 mg, e.g. 1, 5, or 10 mg, or any other intermediate dosage of active agent. Said solid oral dosage forms, e.g. tablets, may be smaller, for a given amount of active agent, than any known formulations of this active agent.

In a first group of compositions, example 2 is excluded. In a second group of compositions example 3 is excluded.

In a further aspect the present invention relates to an oral solid pharmaceutical, e.g. in form of a tablet, of valsartan or a pharmaceutically acceptable salt thereof or hydrate thereof comprising between 10 to 80%, e.g. 20 and 80%, e.g. 25 to 75%, e.g. 30 to 70%, e.g. 35 to 65%, e.g. 40 to 60%, e.g. 50% of a disintegrant based on the total weight of the composition.

In a further aspect the present invention relates to an oral solid pharmaceutical, e.g. in form of a tablet, of valsartan or a pharmaceutically acceptable salt thereof or hydrate and a disintegrant in a weight ratio of e.g. between 10:1 to 0.5:1, e.g. 9.5:1, e.g. 8:1, e.g. between 5:1 to 0.5:1, e.g. 5:1 to 1:1, e.g. 2.9:1 to 1:1, e.g. 2.5 to 1:1, e.g. 2 to 1:1, e.g. 1.5:1.

In a preferred embodiment, the compositions of the invention comprises more than 30% of a filler, e.g. microcrystalline cellulose, by weight based on the total weight of the core components of said solid oral dosage form, e.g. 31 to 65%, e.g. 40 to 60%, e.g. 50%.

Preferably, in the compositions of the invention the active agent and the filler are present in a weight ratio of from 4:1 to 0.3:1, e.g. 3:1 to 0.3:1, e.g. 2.5:1 to 0.5:1, e.g. 2:1 to 1:1, e.g. 1.4:1.

The compositions of the invention may be in the form of tablets, e.g. compressed tablets, which are obtainable by the manufacturing process disclosed below.

The compositions of the invention comprise additives conventional in the dosage form in question. Tabletting aids, commonly used in tablet formulation can be used and reference is made to the extensive literature on the subject, see in particular Fiedler's "Lexikon der Hilfsstoffe", 4th Edition, ECV Aulendorf, 1996, which is incorporated herein by reference. These include but are not limited to disintegrants, binders, lubricants, glidants, stabilising agents, fillers or diluents, surfactants and the like.

As disintegrants suitable for compositions of this invention, one can particularly mention
- carboxymethylcellulose calcium (CMC-Ca),
- carboxymethylcellulose sodium (CMC-Na, croscarmellose sodium), available as e.g. Ac-Di-Sol®, Primellose®, Pharmacel® XL, Exptocel®, and Nymcel® ZSX, e.g. having a molecular weight of 90 000 - 700 000
- crosslinked polyvinyipyrrolidones (PVP), e.g. crospovidones, e.g. Pofyplasdone® XL and Kollidon® CL, in particular having a molecular weight in excess of 1 000 000, more particularly having a particle size distribution of less than 400 microns or less than 74 microns
- alginic acid, sodium alginate and guar gum.

Preferably the disintegrant may be crosslinked PVP, Crospovidone, crosslinked CMC and Ac-Di-Sol®. The most preferred disintegrant is Crospovidone.

As binders suitable for compositions of this invention, one can particularly mention
- starches, e.g. potato starch, wheat starch, corn starch,
- celluloses such as microcrystalline cellulose, e.g. products known under the registered trade marks Avicel®, Filtrak®, Heweten® or Pharrnacel®, hydroxypropyl cellulose, hydroxyethyl cellulose, and hydroxypropylmethyl cellulose, e.g. hydroxypropyl cellulose having a hydroxyrpropyl content of 5 to 16% by weight and a molecular weight of from 80 000 to 1 150 000, more particularly 140 000 to 850 000.

As glidants suitable for compositions of this invention, one can mention in particular
- colloidal silica, e.g. Aerosil®,
- magnesium trisilicate,
- powdered cellulose,
- starch,
- talc, and
- tribasic calcium phosphate.

As fillers or diluents suitable for compositions of this invention, one can mention
- confectioner's sugar, compressible sugar, dextrates, dextrin, dextrose, lactose, mannitol, sorbitol, sucrose
- microcrystalline cellulose, in particular having a density of about 0.45 g/cm³, e.g. Avicel®, or powdered cellulose, and
- talc.
A preferred filler may be Avicel®.

As lubricants suitable for compositions of this invention, one can mention in particular
- magnesium-, aluminium-, or calcium- stearate,
- polyethylene glycol (PEG) having a molecular weight of 4000 to 8000, and
- talc.

One or more of these additives may be selected and used by the skilled artisan having regard to the particular desired properties of the solid oral dosage form by routine experimentation and without any undue burden.

The amount of each type of additive employed, e.g. glidant, binder, disintegrant, filler or diluent and lubricant may vary within ranges conventional in the art. Thus for example, the amount of glidant may vary within a range of from 0.1 to 10% by weight, in particular 0.1 to 5% by weight, e.g. 0.1 to 0.5% by weight; the amount of binder may vary within a range of from about 10 to 65.3% by weight, e.g. 10 to 45%, e.g. 20 to 30% by weight; the amount of disintegrant may vary within a range of 5 to 60% by weight, e.g. 13 to 50%, e.g. 15 to 40%, e.g. 20 to 30%, e.g. 25%; the amount of filler or diluent may vary within a range of from 15 to 65% by weight e.g. 20 to 50%, e.g. 25 to 40%, e.g. 30%, whereas the amount of lubricant may vary within a range of from 0.1 to 5.0% by weight.

It is a characteristic of the present solid oral dosage forms, e.g. tablets, that they contain only a relatively small amount of additives given the high content of active agent. This enables the production of physically small unit dosage forms. The total amount of additives in a given unit dosage may be about 65% or less by weight based on the total weight of the solid oral dosage form, more particularly about 50% or less. Preferably the additive content is in the range of about 35 to 55% by weight, more particularly 45 to 55% by weight, e.g. 38 to 43% by weight.

The absolute amounts of each additive and the amounts relative to other additives is similarly dependent on the desired properties of the solid oral dosage form and may also be chosen by the skilled artisan by routine experimentation without undue burden. For example, the solid oral dosage form may be chosen to exhibit accelerated and/or delayed release of the active agent with or without quantitative control of the release of active agent.

Thus, where accelerated release is desired, e.g. about 90% release within a ten minute, more particularly a five minute period, a disintegrant such as crosslinked polyvinyl pyrrolidone, for example those products known under the registered trade marks Polyplasdone® XL or Kollidon®CL, in particular having a molecular weight in excess of 1 000 000, more particularly having a particle size distribution of less than 400 microns or less than 74 microns, or reactive additives (effervescent mixtures) that effect rapid disintegration of the lablet in the presence of water, for example so-called effervescent tablets that contain an acid in solid form, typically citric acid, which acts in water on a base containing chemically combined carbon dioxide, for example sodium hydrogencarbonate or sodium carbonate, and releases carbon dioxide.

The pharmaceutical compositions of the present invention are useful in the known indications of the particular active agent incorporated therein including lowering of the blood pressure, either systolic or diastolic or both. The conditions for which the instant invention is useful include, without limitation, hypertension (whether of the malignant, essential, reno-vascular, diabetic, isolated systolic, or other secondary type), congestive heart failure, angina (whether stable or unstable), myocardial infarction, artherosclerosis, diabetic nephropathy, diabetic cardiac myopathy, renal insufficiency, peripheral vascular disease, left ventricular hypertrophy, cognitive dysfunction (such as Alzhelmer's) and stroke.

The exact dose of active agent and the particular formulation to be administered depend on a number of factors, e.g. the condition to be treated, the desired duration of the treatment and the rate of release of the active agent. For example, the amount of the active agent required and the release rate thereof may be determined on the basis of known in vitro or in vivo techniques, determining how long a particular active agent concentration in the blood plasma remains at an acceptable level for a therapeutic effect.

For example, the compositions of the invention in clinical trials have a higher bioavailability as compared to the commercial form of Diovan®.

Preferably the drug release rate of the composition of the invention is more than 70% in 10 minutes, above 80%, e.g. 90%, over 30 minutes, and above 95% over 45 minutes, e.g. at a pH range of 4 to 7.2, e.g. at pH 4.5 to 7, e.g. at pH 6.8.

For example dosages in the range of 1 mg to 400 mg of valsartan per day for a 75 kilogram mammal, e.g. humans, and in standard animal models, may be used. An excellent tolerability of valsartan provided by the compositions may be observed in standard animal tests and in clinical trials.

In a further aspect the invention relates to a method of administering valsartan to a subject in need of valsartan treatment which comprises administering to the subject a composition of the invention.

In a further aspect the Invention relates to the use of valsartan as active agent in the manufacture of any composition as hereinabove described.

The invention provides in another of its aspects a process of making a solid oral dosage form, e.g. tablets, as hereinabove described. Such solid oral dosage form may be produced by working up components as in WO 97/49394 (herein incorporated by reference), e.g. as defined hereinabove, in appropriate amounts, to form unit dosage forms.

For example there is provided a process of making the composition of the invention as hereinabove described comprising the steps of
i) grinding the active agent and pharmaceutically acceptable additives,
ii) subjecting a mixture of the ground active agent and additives to compression to form a comprimate (coprimate) (the compacted mass)
iii) converting the comprimate (coprimate) to form a granulate and
iv) compressing the granulate to form the solid oral dosage form.

The process is carried out in the absence of water, i.e, it is a dry compression method. The process may be carried out under ambient conditions of temperature and humidity; it is not necessary to ensure that the process is carried out in a dry atmosphere.

The initial grinding step i) may be carried out according to conventional milling methods or micronisation methods.

The active agent and the additives can be milled either individually or together to particle sizes from about 0.1 micrometers (µ) to about 1500 µ, e.g. 1.0 µ to 900 µ, e.g. 60µ to 600 µ. At least 90 % of the crystals of both the active agent and the additives are present in these ranges. Particles of this size are obtained by conventional methods, e.g. grinding in an air jet mill, hammer and screen mill, fine impact mill, ball mill or vibrator mill.

Micronisation is preferably effected by known methods using an ultrasonic disintegrator, e.g. of the BRANSON Sonifier type, or by stirring a suspension with a high speed agitator, for example with a stirrer of the HOMOREX type.

The ground particles may optionally at this stage be sieved and mixed according to known methods.

Compression to form a comprimate (coprimate) requires the compaction of the dry ground components. Compaction may be carried out using a slugging technique or preferably, roller compaction. Roller compaction apparatus is conventional and essentially utilises two rollers which roll towards each other. A hydraulic ram forces one of the rollers against the other to exert a compacting force against the ground particles fed into the roller compactor via a screw conveyor system.

A compaction force of between 25 and 65 kN, e.g. 25 and 45 kN may be used. Within this range of compaction forces it has surprisingly been found that for each particular formulation a minimum compaction force should be used in order to obtain a solid oral dosage form wherein the granulate disintegrates into discrete primary particles at a desirable rate, e.g. disintegration occurs approximately six times faster for a solid oral dosage form compressed above a minimum compaction force. Such a rapid disintegration rate is unusual for tablets and is similar to the disintegration rate of a capsule formulation. The particular minimum compaction force is dependent on the active agent content in any given formulation and therefore also depends on the amount and nature of the additives present.

Given this Information, the skilled addressee is clearly able to determine the minimum compaction force for other formulations using routine experimentation and without undue burden.

The roller speed may be set at between 1 and 20 rpm and preferably 9 to 15 rpm. After passing through the rollers the compacted mass (the comprimate (coprimate)) resembles a thin ribbon in segments.

The comprimate (coprimate) may be screened and or milled to produce the granulate. Screening in its simplest form involves the passing of the comprimate (coprimate) emerging from the rollers through a sieve under mechanical pressure. More preferably, the comprimate (coprimate) is screened using an oscillating or rotating mill, e.g. a MGI 624 Frewitt (Key International Inc.).

The compression of the granulates to tablet cores can be carried out in a conventional tabletting machine, e.g. in an EK-0 Korsch eccentric tabletting machine or a rotary compression machine, e.g. at a compression greater than 2 kN. The tablet cores may vary in shape and be, for example, round, oval, oblong, cylindrical or any other suitable shape, and may also vary in size depending on the concentration of the therapeutic agents. A characteristic of tablets according to the invention is their small size having regard to the amount of active agent contained therein.

In a preferred embodiment of the invention tablets obtained by the compression method described above are slightly oval in lateral and/or longitudinal cross-section. The edges of the tablets may be bevelled or rounded.

In a particularly preferred embodiment of the invention a solid oral dosage form is compressed in the form of a tablet having an oblong shape in which the ratio of dimensions length : width : height is e.g. 2.5-5.0 : 0.9-2.0 : 1.0, e.g. 2.86-3.16 : 1.1-1.3 : 1.0, e.g. 14.0-14.2mm : 5.5-5.7mm: 4.5-4.9mm and preferably in which the base and top face of the tablet independently of one another are planar or convexly curved about the longitudinal axis; the side faces are planar, the end faces can be of any shape and the edges are optionally bevelled or rounded.

In a particularly preferred embodiment of the invention a solid oral dosage form is compressed, from the granulate, in the form of a tablet, e.g. containing 40 mg or 80 mg valsartan, which is essentially disc-shaped with the upper and lower faces having a slightly convex surface. Preferably the tablet has a diameter of about 6 to 6.5 mm and a depth of about 2.5 to 3.5 mm, or a diameter of about 8 to 8.5 mm and a depth of about 3 to 4 mm.

In another particularly preferred embodiment of the invention a solid oral dosage form is compressed, from the granulate, in the form of a tablet, e.g. containing 160 mg valsartan, of oblong shape in which the length is approximately 10.0 to 15.0 mm, the width is approximately 5.0 to 6.0 mm, and the height is approximately 3 to 6 mm, e.g. 3.0 to 4.0 mm.

In another particularly preferred embodiment of the invention a solid oral dosage form is compressed, from the granulate, in the form of a tablet, e.g. containing 80, 160, or 320 mg valsartan, of an almond shape in which the length Is approximately 9 to 11 mm, the width is approximately 5 to 6.5 mm at its widest point, and the height is approximately 3 to 4 mm, or in which the length is approximately 12 to 14 mm, the width Is approximately 7 to 8 mm at its widest point, and the height is approximately 4 to 5 mm, or in which in which the length is approximately 15 to 17 mm, the width is approximately 9 to 10 mm at its widest point, and the height is approximately 5 to 6.5 mm.

In another particularly preferred embodiment of the invention a solid oral dosage form is compressed, from the granulate, in the form of a tablet, e.g. containing 320 mg valsartan, of an almond shape in which the length is approximately 15 to 17 mm, the width is approximately 9 to 10 mm at its widest point, and the height is approximately 5 to 7 mm.

In yet another preferred embodiment of the invention there is provided a tablet which is essentially disc-shaped with the upper and lower faces having a slightly convex surface. Preferably the tablet has a diameter of about 8 to 8.5 mm and a depth of about 3 to 3.5 mm, or a diameter of about 16 mm and a depth of about 6 mm. The tablets may occupy a volume from about 0.1 cm³ to about 1 cm³, e.g. 0.1 cm³ to about 0.45 cm³, e.g. 0.2 to 0.3 cm³, e.g. about 0.125 cm³ or 0.25 cm³.

They may furthermore be transparent, colourless or coloured and also marked so as to impart to this product an individual appearance and to make them instantly recognizable. The use of dyes can serve to enhance the appearance as well as to identify the compositions. Dyes suitable for use in pharmacy typically include carotinoids, iron oxides or chlorophyll.

Following is a description by way of example only of compositions of this invention.

**Examples 1 to 4 :**

| | **1** | **2** | **3** | **4** |
|---|---|---|---|---|
| **Components** | **COMPOSITION PER UNT (mg)** | **COMPOSITION PER UNT (mg)** | **COMPOSITION PER UNIT (mg)** | **COMPOSITION PEIR UNG (mg)** |
| **Granulation** | | | | |
| Diovan Drug Substance | 20.0 | 40.0 | 80.0 | 320.0 |
| Hydrochlorothiazide Drug Substance | | --- | | --- |
| Microcrystalline Cellulose (NF, Ph.Eur.)/ Avicel PH 102 | 62.0 | 124.0 | 54.0 | 216.0 |
| Crospovidone (NF, Ph.Eur.) | 10.0 | 20.0 | 20.0 | 80.0 |
| Colloidal Anhydrous Silica (Ph. Eur.)/Colloidal Silicon Dioxide (NF)/Aerosll 200 | 0.5 | 1.0 | 0.75 | 3.0 |
| Magnesium Slearate (NF, Ph.Eur.) | 1.0 | 2.0 | 2.5 | 10.0 |
| **Blending** | | | | |
| Colloidal Anhydrous Silica (Ph. Eur.)/Colloldal Silicon Dioxide (NF)/Aerosil 200 | 0.5 | 1.0 | 0.75 | 3.0 |
| Magnesium Stearate, NF, Ph.Eur. | 1.0 | 2.0 | 2.0 | 8.0 |
| **Core Weight/Batch Weight** | 95.0/47.5kg | 190.0/47.5kg | 160.0/48.0kg | 640.0/73.5kg |

### Example 5:

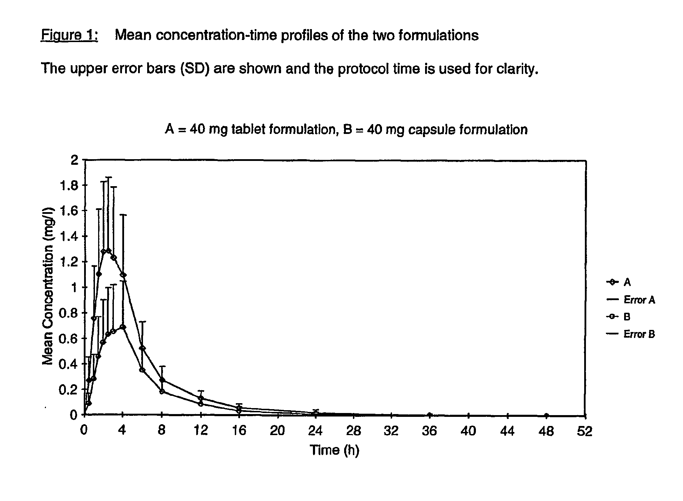

**Table 1. (re. Fig 1) Summary analysis of least squares means for valsartan pharmacokinetic parameters (all completed subjects with evaluable parameters on both treatments)**

| Parameter | 40 mg tablet (Treatment A) Least squares mean^{a}(N) | 40 mg capsule Reference (Treatment B) Least squares mean^{a} (N) | Ratio of least square means^{b} | 90% confidence interval for ratio of least squares means^{c} |
|---|---|---|---|---|
| AUCall | 6.732 (60) | 3.922 (60) | 1.72 | (1.49, 1.97) |
| AUCinf | 6.859 (60) | 4.037 (60) | 1.70 | (1.48, 1.95) |
| Cmax | 1.245 (60) | 0.681 (60) | 1.83 | (1.57, 2.13) |

| | | | | |
|---|---|---|---|---|
| N represents the number of observations including repeated dosing. ^{a} The least squares means are expressed on the original (anti-log) scale ^{b} The ratio of means on the original scale is estimated by the antilog of the difference in least squares means on the log scale. ^{c} The confidence interval for the ratio of the tablet to capsule on the original scale is obtained by taking the anti-logs of the confidence limits for the difference of the treatment least squares means on the log scale. | | | | |

40 mg Diovan® capsule as marketed:
inner phase: valsartan 40.0 mg; microcrystalline cellulose/Avicel PH 102: 12.55 mg;
polyvinylpyrrolidone K30: 6.25 mg; sodium lauryl sulphate: 0.3 mg;
outer phase: crospovidone: 6.5 mg; magnesium stearate: 0.65 mg
total weight: 66.25 mg; capsule size: 3

### Example 6:

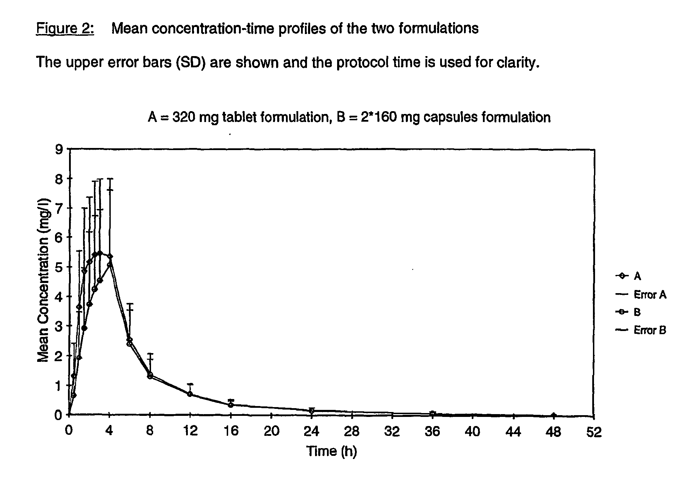

**Table 2. (Re. Fig 2) Summary analysis of least squares means for valsartan pharmacokinetic parameters (all completed subjects with evaluable parameters on both treatments)**

| Parameter | 320 mg tablet (Treatment A) Least squares mean^{a} (N) | 2*160 mg capsules Reference (Treatment B) Least squares mean^{a} (N) | Ratio of least square means^{b} | 90% confidence interval for ratio of least squares means^{c} |
|---|---|---|---|---|
| AUCall | 36.53 (60) | 29.3 (60) | 1.24 | (1.14, 1.35) |
| AUCinf | 37.32 (60) | 30.17 (60) | 1.24 | (1.14, 1.35) |
| Cmax | 6.23 (60) | 4.88 (60) | 1.28 | (1.15, 1.41) |

| | | | | |
|---|---|---|---|---|
| N represents the number of observations including repeated dosing. ^{a} The least squares means are expressed on the original (anti-log) scale ^{b} The ratio of means on the original scale is estimated by the antilog of the difference In least squares means on the log scale. ^{c} The confidence interval for the ratio of the tablet to capsule on the original scale is obtained by taking the anti-logs of the confidence limits for the difference of the treatment least squares means on the log scale. | | | | |

160 mg Diovan® capsules as marketed:
inner phase: valsartan 160.0 mg; microcrystalline cellulose/Avicel PH 102: 50.2 mg;
polyvinylpyrrolidone K30: 125.0 mg; sodium lauryl sulphate: 1.2 mg;
outer phase: crospovidone: 26.0 mg; magnesium stearate: 2.6 mg.
total weight: 265.0 mg; capsule size: 1--

### Example 7 :

**Table 3. Pharmacokinetic parameters of the 40 mg tablet formulation and the 40 mg capsule formulation (marketed)**

| Formulation | Statistics | Tₘₐₓ (h) | Cₘₐₓ (mg/l) | AUCₗₐₛₜ (h.mg/l) | AUCₐₗₗ (h.mg/l) | AUC₀₋₈ (h.mg/l) |
|---|---|---|---|---|---|---|
| Tablet | N¹⁾ = 61 | | | | | |
| | Mean | 2.32 | 1.425 | 7.514 | 7.719 | 7.836 |
| | SD | 0.75 | 0.578 | 2.960 | 2.992 | 3.024 |
| | Min | 1.50 | 0.152 | 0.806 | 0.922 | 1.104 |
| | Median | 2.00 | 1.284 | 7.131 | 7.346 | 7.502 |
| | Max | 4.03 | 3.363 | 15.637 | 15.893 | 16.192 |
| | CV% | 32.2 | 40.5 | 39.4 | 38.8 | 38.6 |
| | Geom Mean | 2.21 | 1.301 | 6.861 | 7.073 | 7.202 |
| | LL of 95% Cl | 2.12 | 1.277 | 6.756 | 6.952 | 7.062 |
| | UL of 95% Cl | 2.51 | 1.573 | 8.272 | 8.485 | 8.611 |
| | | | | | | |
| Capsule | N = 60 | | | | | |
| | Mean | 3.32 | 0.760 | 4.190 | 4.347 | 4.461 |
| | SD | 0.99 | 0.386 | 2.132 | 2.190 | 2.227 |
| | Min | 1.50 | 0.072 | 0.472 | 0.472 | 0.472 |
| | Median | 4.00 | 0.741 | 4.076 | 4.244 | 4.351 |
| | Max | 6.03 | 1.863 | 9.785 | 10.018 | 10.140 |
| | CV% | 29.8 | 50.8 | 50.9 | 50.4 | 49.9 |
| | Geom Mean | 3.17 | 0.653 | 3.588 | 3.733 | 3.843 |
| | LL of 95% Cl | 3.07 | 0.660 | 3.639 | 3.781 | 3.885 |
| | UL of 95% Cl | 3.58 | 0.860 | 4.741 | 4.913 | 5.036 |

### Example 8:

**Table 4 Summary of the pharmacokinetic parameters of the 320 mg tablet formulation and the 2*160 mg capsule formulation (marketed)**

| Formulation | Statistics | Tₘₐₓ (h) | Cₘₐₓ (mg/l) | AUCₗₐₛₜ (h.mg/l) | AUCₐₗₗ (h.mg/l) | AUC₀₋₈ (h.mg/l) |
|---|---|---|---|---|---|---|
| Tablet | N = 60 | | | | | |
| | Mean | 2.86 | 6.509 | 37.77 | 38.24 | 39.18 |
| | SD | 0.92 | 2.673 | 14.90 | 14.86 | 15.29 |
| | Min | 1.00 | 2.41 | 15.08 | 15.08 | 16.21 |
| | Median | 3.00 | 6.07 | 35.65 | 35.65 | 37.32 |
| | Max | 4.17 | 14.09 | 83.88 | 83.88 | 86.84 |
| | CV% | 32.3 | 41.1 | 39.4 | 38.9 | 39.0 |
| | Geom Mean | 2.70 | 6.032 | 35.10 | 35.60 | 36.45 |
| | LL of 95% Cl | 2.62 | 5.819 | 33.93 | 34.40 | 35.22 |
| | UL of 95% Cl | 3.10 | 7.200 | 41.62 | 42.08 | 43.13 |
| | | | | | | |
| Capsule | N = 60 | | | | | |
| | Mean | 3.28 | 5.534 | 32.29 | 32.74 | 33.51 |
| | SD | 0.99 | 2.545 | 14.09 | 14.05 | 14.20 |
| | Min | 1.00 | 2.05 | 11.29 | 11.59 | 11.73 |
| | Median | 3.01 | 4.73 | 29.45 | 30.05 | 30.9 |
| | Max | 6.00 | 11.81 | 73.81 | 73.81 | 75.26 |
| | CV% | 30.3 | 46.0 | 43.6 | 42.9 | 42.4 |
| | Geom Mean | 3.12 | 4.998 | 29.48 | 29.98 | 30.72 |
| | LL of 95% Cl | 3.02 | 4.876 | 28.65 | 29.1 | 29.84 |
| | UL of 95% Cl | 3.54 | 6.191 | 35.93 | 36.37 | 37.18 |

## Claims

1. An oral solid pharmaceutical composition comprising pharmacologically effective amounts of valsartan or a pharmaceutically acceptable salt thereof or hydrate thereof and at least one pharmaceutically acceptable excipient and which is, on average, at least 1.2 times more bioavailable than a valsartan capsule.

2. A composition according to claim 1 which is, on average, at least 1.5 times more bioavailable than a valsartan capsule when administered as a dose of 40 mg in a single dose human bioavailability study.

3. A composition according to claim 1 which is, on average, at least 1.2 times more bioavailable than a valsartan capsule when administered as a dose of 320 mg in a single dose human bioavailability study.

4. An oral solid pharmaceutical composition comprising pharmacologically effective amounts of valsartan or a pharmaceutically acceptable salt thereof or hydrate thereof and at least one pharmaceutically acceptable excipient and having an AUC higher than 4.5 h.mg/l at same conditions as a 40mg capsule has an AUC of 3.9 h.mg/l.

5. An oral solid pharmaceutical composition comprising pharmacologically effective amounts of valsartan or a pharmaceutically acceptable salt thereof or hydrate thereof and at least one pharmaceutically acceptable excipient and having an AUC higher than 30 h.mg/l at same conditions as a 320mg capsule has an AUC of 29.4 h.mg/l.

6. An oral solid pharmaceutical composition comprising pharmacologically effective amounts of valsartan or a pharmaceutically acceptable salt thereof or hydrate thereof and at least one pharmaceutically acceptable excipient and having a Cmax of about at least 0.77 mg/l when administered as a dose of 40 mg in a single dose human bioavailability study.

7. An oral solid pharmaceutical composition comprising pharmacologically effective amounts of valsartan or a pharmaceutically acceptable salt thereof or hydrate thereof and at least one pharmaceutically acceptable excipient and having a Cmax of about at least 4.75 mg/l when administered as a dose of 320 mg in a single dose human bioavailability study.

8. An oral solid pharmaceutical composition of valeartan or a pharmaceutically acceptable salt thereof or hydrate thereof comprising between 20% and 80% of a disintegrant based on the total weight of the composition.

9. A composition according to claim 8 wherein the valsartan or a pharmaceutically acceptable salt thereof or hydrate and the disintegrant are in a weight ratio of between 5.1:1 and 0.5:1.

10. A composition according to any preceding claim in a compressed form.

11. A method of administering valsartan to a subject in need of valsartan treatment which comprises administering to the subject a composition according to any of the preceding claims.

12. Use of valsartan as active agent in the manufacture of a composition according to any of the preceding claims.
